# EUROPEAN PATENT APPLICATION

(11) **EP 4 763 076 A1**
(43) Date of publication of application: **24.06.2026**
(21) Application number: 24222959.9
(22) Date of filing: 23.12.2024
(51) Int. Cl.: A61B 5/11, A61B 5/00

(54) **SYSTEM AND METHOD CONFIGURED FOR IDENTIFYING, MONITORING, PREDICTING AND/OR DETECTING TRAUMATIC BRAIN INJURIES IN AN ACTIVE ENVIRONMENT**

(71) Applicant: Contego Sports Limited, Oranmore, County Galway (IE)
(72) Inventor: GANLY, MARK, Galway (IE); FOX, FRANK, Galway (IE); GANLY, SANDRA, Galway (IE)
(74) Representative: White, Jonathan Patrick

(57) **Abstract**

The present invention relates to a system and method configured for identifying, monitoring, predicting and/or detecting brain injury to a human participant in an active environment. The system comprises at least one physiological sensor communicatively coupled to the participant to measure one or more physiological characteristics of the participant, the at least one physiological sensor configured to transmit data relating to the measured one or more physiological characteristics as physiological data, at least one biomechanical sensor communicatively coupled to the participant to measure impact forces experienced by the participant, the at least one biomechanical sensor configured to transmit data relating to the measured impact forces as impact force data, means for processing the impact force data and physiological data to determine whether a first alert criteria for a head injury risk event is met, and means for outputting a substantially instantaneous alert or notification to one or more remote computing devices if the first alert criteria is met. Also provided is traumatic brain injury indicator database configured with algorithmic means for analysing data in a profile record to determine whether a second alert criteria is met, the second alert criteria relating to an updated cumulative head injury or mild traumatic brain injury risk for the participant.

## Description

The present invention relates to a system and method configured for identifying, monitoring, predicting and/or detecting traumatic brain injuries, including concussions, other mild traumatic brain injuries, and cumulative damage resulting from such injuries in human participants, and particularly, although not limited to, human participants in active environments including those engaged in sporting activities, industrial settings and/or the military.

In sporting activities, such as rugby, soccer, boxing, mixed martial arts, American football etc traumatic brain injuries (TBI), including concussions and mild traumatic brain injuries (mTBls), are a significant concern for player safety. Similar issues of concern exist in industrial settings, the military and other active environments.

Currently, in many sports a standard-of-care exists where observers/players are aware of events likely to have caused mTBls and are aware of the symptoms of an mTBI. In such sports, the affected players are removed from play for a head injury assessment (HIA). Failing the HIA results in removal of the player from the game and a return-to-play protocol is typically followed over the following weeks.

However, research indicates that 90% of athletes who sustain a concussion do not exhibit any immediate, observable symptoms, making it difficult to diagnose these injuries in real time.

These hidden concussions, along with the cumulative effects of smaller, sub-concussive impacts, may lead to long-term neurological conditions, such as dementia, reduced cognitive functioning, and chronic traumatic encephalopathy (CTE). Athletes who continue to play, even for a few minutes after sustaining a concussion, may take twice as long to recover as those who come off the field of play immediately.

Current diagnostic tools thus only become useful after visible symptoms emerge or after a suspected event has occurred. This results in delayed interventions and increases the risk of long-term damage from repeated, undiagnosed head impacts.

For example, in the game of rugby, recent developments have seen the introduction of instrumented mouthguards (IMGs) at the higher levels of the sport. When an impact over a specific threshold is measured, that player is removed from play for a HIA and subsequent treatment.

Such an approach to player safety has many limitations including:
- The player must be wearing a functioning instrumented mouthguard.
- The over-threshold impact must be noticed and acted upon.
- The threshold is a universal value without consideration of the individual's characteristics.
- The player may have suffered multiple sub-threshold impacts causing cumulative injury which will not be flagged.

Accordingly, identifying, monitoring, predicting and/or detecting concussions and other TBls early, particularly in cases where traditional symptom-based diagnostics are insufficient is of great importance to player safely.

Moreover, there is a need to improve the way in which cumulative damage relating to concussions and other TBIs in players is identified, monitored, and/or detected.

It is therefore an object of the present invention to provide a system and method that goes at least some way toward overcoming the above problems and/or which will provide the public and/or industry with a useful alternative.

Further aspects of the present invention will become apparent from the ensuing description which is given by way of example only.

According to the invention, there is provided a system configured for identifying, monitoring, predicting and/or detecting brain injury to a human participant in an active environment, the system comprising:
at least one physiological sensor communicatively coupled to the participant to measure one or more physiological characteristics of the participant, the at least one physiological sensor configured to transmit data relating to the measured one or more physiological characteristics as physiological data,
at least one biomechanical sensor communicatively coupled to the participant to measure impact forces experienced by the participant, the at least one biomechanical sensor configured to transmit data relating to the measured impact forces as impact force data,
means for processing the impact force data and physiological data to determine whether a first alert criteria for a head injury risk event is met, and
means for outputting a substantially instantaneous alert to one or more remote computing devices if the first alert criteria is met.

The present invention provides a multi-sensor system that combines biomechanical and physiological monitoring to improve TBI detection of participants in an environment having risks associated with head injuries, such as a sporting activity, an industrial setting and/or a military setting.

By correlating impact forces with physiological responses, the invention provides a improvement in the assessment of head injuries. A combination of sensors to capture impact data and physiological data relating to measured body responses thus enhances the detection and analysis of concussive and sub-concussive events in near real time.

When the combination of impact force data and physiological data meet a first alert criteria for a head injury risk event, a substantially instantaneous alert is outputted at a computer device coupled with or connected to the system, and the alert makes coaches, medical and other relevant staff aware that a head injury risk event meeting the first alert criteria has occurred so that the participant may be removed from the active environment and receive the appropriate care and assessment.

The alert may be an audible or visible alert, and may be in the form of a device screen message, an SMS, email, or any other type of alert capable of relaying that the first alert criteria for a head injury risk event has occurred and action should be taken, such as removing the participant from the active environment for assessment or whatever intervention is deemed necessary by coaching and medical staff.

Preferably, the at least one biomechanical sensor is one or more or a combination of: a force sensitive resistor sensor, an accelerometer sensor and/or a gyroscope sensor.

Preferably, the at least one physiological sensor is one or more or a combination of: an Electroencephalography (EEG) sensor, a Transcranial Doppler (TCD) sensor, an electrocardiography (ECG) sensor, a Photoplethysmography (PPG) sensor, a Galvanic skin response (GSR) sensor, a heart rate sensor and derivates, and/or a body temperature sensor.

Preferably, the at least one physiological sensor and the at least one biomechanical sensor are integrated in headgear worn by the participant.

Integrating advanced biomechanical and physiological sensors into smart headgear provides a suitable wearable article for locating sensors on or about the head to detect and monitor head injury caused by impacts.

Preferably, the means for processing the impact force data and physiological data comprises means for comparing the impact force data and physiological data with a first alert criteria threshold level, whereby if the impact forces corresponding to the impact force data and the physiological characteristics corresponding to the physiological data are determined to be equal to or exceed the first alert criteria threshold level then the first alert criteria is determined to be met.

Preferably, the system comprises a traumatic brain injury indicator database configured with a profile record for the participant and impact force data and physiological data are recorded in the profile record.

Preferably the traumatic brain injury indicator database is configured with algorithmic means for analysing data in the profile record to determine whether a second alert criteria is met, the second alert criteria relating to cumulative head injury or mTBI risk for the participant.

The algorithmic means may be provided by an artificial intelligence (Al) driven algorithm and/or machine learning algorithm.

Preferably, the profile record for the participant is configured with historical data, including impact force data and physiological data recorded for previous head injury risk events and personal characteristics including biomarkers and other physiological data relating to the participant.

Preferably, the traumatic brain injury indicator database is configured with data records corresponding to histories of clinically validated injured and non-injured participants.

Preferably, the system comprises means to compare data in the participant's profile record with a second alert criteria threshold level computed from records of data for clinically validated injured and non-injured participants, whereby if the second alert criteria threshold level is equalled or exceeded then a second alert criteria is deemed to be met and a substantially instantaneous alert is transmitted to the at least one remote computing device.

Thus, a second alert criteria will indicate an updated elevated risk of cumulative head injury or mTBI.

According to the invention, there is provided a method configured for identifying, monitoring, predicting and/or detecting brain injury to a human participant in an active environment, the method comprising steps of:
measuring one or more physiological characteristics of the participant by at least one physiological sensor communicatively coupled to the participant and transmitting data relating to the measured one or more physiological characteristics as physiological data,
measuring impact forces experienced by the participant by at least one biomechanical sensor communicatively coupled to the participant and transmitting data relating to the measured impact forces as impact force data,
processing the impact force data and physiological data to determine whether a first alert criteria for a head injury risk event is met, and
outputting a substantially instantaneous alert to one or more remote computing devices if the first alert criteria is met.

Preferably, the step of processing the impact force data and physiological data comprises comparing the impact force data and physiological data with a first alert criteria threshold level, whereby if the impact forces corresponding to the impact force data and the physiological characteristics corresponding to the physiological data are determined to be equal to or exceed the first alert criteria threshold level then the first alert criteria is determined to be met.

Preferably, the method comprises a step of configuring a traumatic brain injury indicator database configured with a profile record for the participant, in which the impact force data and physiological data are recorded in the profile record.

Preferably, the method comprises a step of analysing data in the profile record using algorithms to determine whether a second alert criteria is met, the second alert criteria relating to cumulative head injury or mTBI risk for the participant.

Preferably, the method comprises a step of configuring the profile record for the participant with historical data, including impact force data and physiological data recorded for previous head injury risk events and personal characteristics including biomarkers and other physiological data relating to the participant.

Preferably, the method comprises a step of configuring the traumatic brain injury indicator database with data records corresponding to histories of clinically validated injured and non-injured participants.

Preferably, the method comprises a step of comparing data in the participant's profile record with a second alert criteria threshold level computed from records of data for clinically validated injured and non-injured participants, whereby if the second alert criteria threshold level is equalled or exceeded then a second alert criteria is determined to be met and a substantially instantaneous alert is transmitted to the at least one remote computing device.

The present invention provides a system and method for identifying, monitoring, predicting and/or detecting concussions and other mild traumatic brain injuries and related conditions that may be caused by activities where the participant suffers single or multiple impacts which generate sensor readings and other data that, in combination with each other and with the personal physiological characteristics and history of the participant, and in combination with the known profiles and histories of other clinically validated injured and non-injured players, consist of a diagnostic adjunct to detect, predict and ultimately prevent concussions and other mTBls and related conditions.

The invention will be more clearly understood from the following description of some embodiments thereof, given by way of example only, with reference to the accompanying drawings in which:
Figs. 1 and 2 are hybrid data flow and block diagrams showing respective first and second parts of a system architecture configured according to the invention,
Fig. 3 is a block entity relationship diagram showing a configuration for a traumatic brain injury indicator database according to the invention, and
Figs. 4 and 5 are process flow diagrams showing respective first and second parts of a method configured according to the invention.

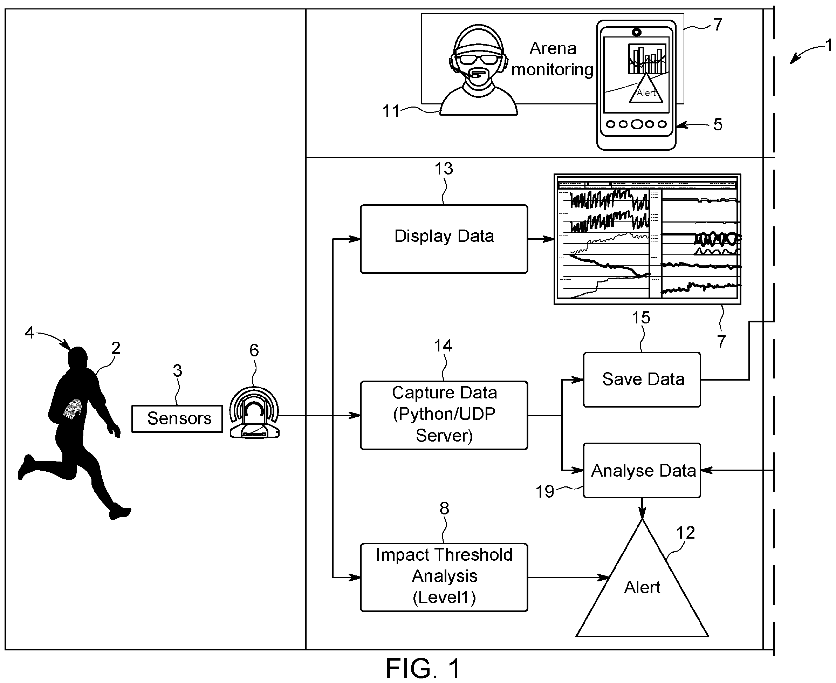

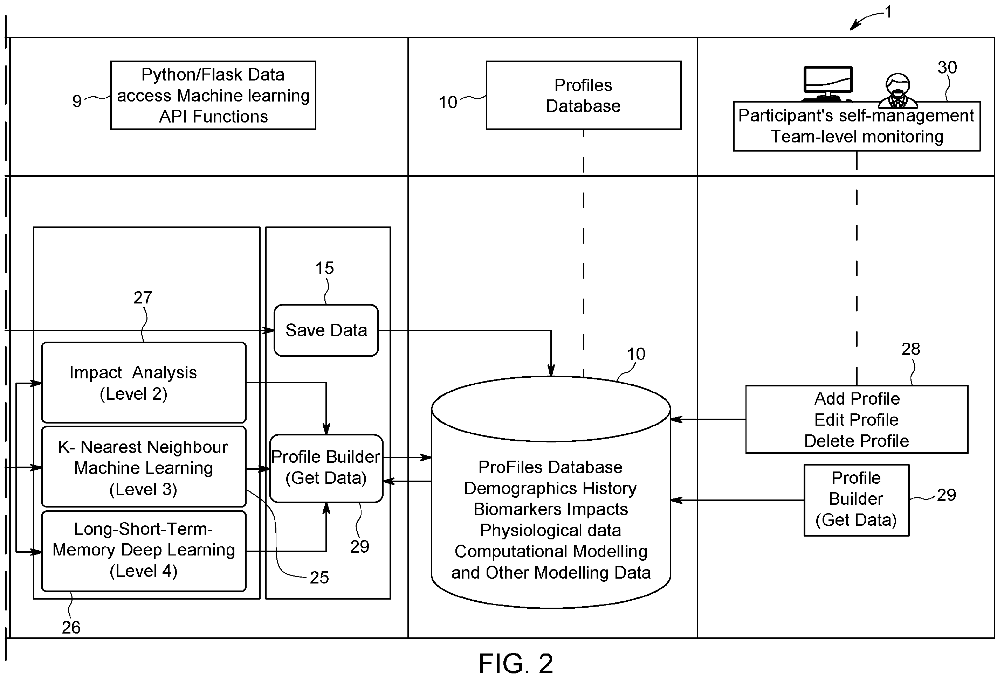

Referring to the drawings, and initially to Figs.1 and 2, there is provided a system 1 configured for identifying, monitoring, predicting and/or detecting brain injury to a human participant 2 in an active environment.

The active environment may be provided by a sporting activity, an industrial setting and/or a military setting. In the context of a sporting activity, it will be understood that a head injury may be brought about by an impact occurring when the head of a participant collides with another player's body or the ground. In an industrial setting, such an impact might occur when the head of the participant is impacted causing injury due to a work-related incident or environment. In a military setting a participant's head might be impacted by an incident or environment.

Accordingly, the application of the present invention is not limited to sport only and has practical utility in a variety of real-life settings, and reference to sport in the following should in no way be seen as limiting.

The system 1 comprises at least one biomechanical sensor, indicated by the reference numeral 3, communicatively coupled to the participant 2 to measure impact forces being experienced by the head of the participant 2.

The one or more biomechanical sensors 3 are configured to transmit via a network 6 data relating to the measured impact forces as impact force data. The network 6 may be configured as a typical computer network for data communication, and may be wired or wireless etc.

The one or more biomechanical sensors 3 may include one or more force sensitive resistors, accelerometers and gyroscopes, to measure impact forces directly. It will be understood that such sensor devices quantify linear and rotational accelerations experienced by the head of the participant during impacts, helping to identify dangerous forces that could cause brain injury.

The system 1 further comprises at least one physiological sensor 3 communicatively coupled to the participant 2 to measure one or more physiological characteristics of the participant 2.

The at least one physiological sensor 3 may be one or more or a combination of: an Electroencephalography (EEG) sensor, a Transcranial Doppler (TCD) sensor, an electrocardiography (ECG) sensor, a Photoplethysmography (PPG) sensor, a Galvanic skin response (GSR) sensor, a heart rate sensor or derivatives, and/or a body temperature sensor.

EEG sensors are widely used in clinical and research settings due to its ability to detect immediate electrical abnormalities caused by brain trauma.

Transcranial Doppler (TCD) sensors detect mTBI through the measurement of blood flow velocity in cerebral vessels. TCD is a non-invasive technique using ultrasound to assess cerebral circulation disruptions, which may provide immediate insights following a head impact.

For monitoring the heart and autonomic nervous system (ANS), electrocardiography (ECG) sensors provide heart rate variability (HRV) analysis by recording the electrical activity and rhythm of the heart through electrodes placed on the skin.

Photoplethysmography (PPG) sensors measure blood flow using light sources and photodetectors. Galvanic skin response (GSR) measure physiological arousal and stress associated with mTBI. GSR measures the electrical conductance of the skin, which varies with moisture level due to sweat gland activity. As a proxy for autonomic nervous system activity, GSR reflects changes in sympathetic nervous system activation. Although it does not directly measure mTBI, it provides valuable insights into stress responses potentially related to brain trauma.

The at least one physiological sensor 3 and the at least one biomechanical sensor 3 for generating kinematic and physiological data are integrated in protective headgear or headwear 4 worn by the participant 2.

The integration of sensors 3 into headgear 4 wearable by the participant 2 requires careful consideration to ensure they do not compromise the comfort or fit of the protective equipment. The sensors 3 are integrated to ensure that their connections withstand impacts, sweat, rain, and dirt. Moreover, ensuring accurate and stable data is important in a high-movement, physically demanding environment. The headgear 4 must be designed so that sensors 3 remain firmly in place without interfering with the participants 2 performance or safety. Additionally, the likelihood of frequent wear and tear in contact sports necessitates that these sensors 3 are easy to maintain and replace.

However, in alternative embodiments the at least one physiological sensor 3 and the at least one biomechanical sensor 3 may not be integrated in headwear but be provided as wearable sensors which are directly fixed to the participant's head. In a further alternative, such sensors 3 may be provided in another wearable article, such a gumshield or clothing article. Accordingly, reference to the sensors 3 being integrated in protective headwear should not be seen as limiting.

The system 1 comprises means 5 for processing the impact force data and physiological data. Such means 5 may be provided by an application executing on one or more computer devices 7 having display means 13 for showing received sensor data, which may be operated or monitored by a coach or medical staff 11.

The means 5 is configured to determine whether the impact force data and physiological data has reached a preliminary level to be recorded. If it has reached this preliminary recordable level, then a determination is made whether a first alert criteria for a head injury risk event is met.

The means 5 comprises or is coupled with means 8 for comparing the impact force data and physiological data with a first alert criteria threshold level, whereby if the impact forces corresponding to the impact force data and the physiological characteristics corresponding to the physiological data are determined to be equal to or exceed the first alert criteria threshold level then a first alert criteria is determined to be met and a head injury risk event is thereby deemed to have occurred.

The means 8 for comparing the impact force data and physiological data with a first alert criteria threshold level may utilise or be coupled with data mining or analytics components to assist in determining the likelihood that a participant has sustained a neurological injury. Such components, shown in Fig. 2 by the reference numeral 9, may be utilised or trained by machine learning or Al algorithms to determine values for the first alert criteria threshold level for the participant.

Additionally, image data, video data, and/or other forms of data, such as computational modelling data or other modelling data, may be used in conjunction with sensor data to determine if the first alert criteria for a head injury risk event has been met.

If the first alert criteria is met then a substantially instantaneous alert, indicated generally by the reference numeral 12, is outputted to the one or more remote computing devices 7, thereby indicating that a head injury risk event has occurred and action should be taken. In the context of a sporting activity such action might include removing the participant from the active environment for assessment or whatever intervention is deemed necessary by coaching and medical staff 11.

The alert 12 may be provided at the computer device 7 as an audible or visible alert and may be in the form of a device screen message, an SMS, email, or any other type of alert capable of relaying that the first alert criteria has been met.

All sensor data and any available image data, video data, and/or other forms of data, such as computational modelling data and or other modelling data, are captured at a computer server 14 and saved at module 15.

As shown in Fig. 2, the system 1 further comprises a traumatic brain injury indicator database 10 configured with a profile record 20 for each participant 2. The impact force data and physiological data derived from the at least one physiological sensor 3 and the at least one biomechanical sensor 3 are recorded in the profile record 20, together with relevant image data, video data, and/or as computational modelling data or other modelling data.

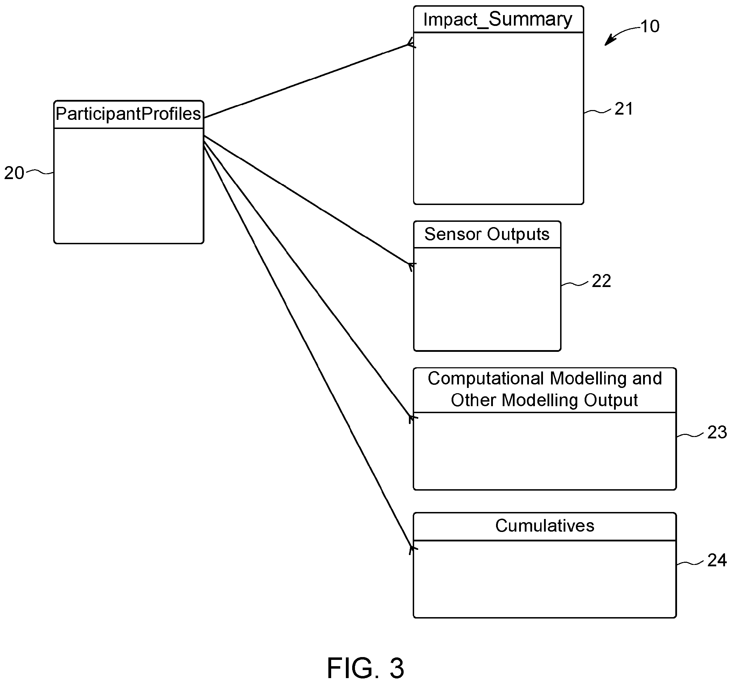

The data set for the traumatic brain injury indicator database 10 is represented by the entity relationship diagram of Fig. 3. In the exemplary arrangement shown, each Participant profile record 20 may have data fields including ID, Age, Gender, Cumulative Playing Time, Cumulative Impacts (g), Cumulative Impacts (n) and Biomarkers. Associated with each Participant profile record 20 may be:
- an Impact Summary record 21 with fields including ID, Device Serial Number, Persisted Event Number, True Positive, Peak Linear Acceleration, Peak Angular Acceleration, Peak Linear Velocity, Peak Angular Velocity, Workload, Location, Date/Time and Quality,
- A Sensor Outputs record 22 with fields including ID, Impact Metrics, Force Metrics, PPG Metrics, GSR Metrics, and HR Metrics,
- a Computational Modelling Output record 23 with fields including ID, Model Inputs for location and Magnitude, Model Outputs for Peak Stress & Strains and Model Output for Images, other modelling output and
- a Cumulative record 24 with fields including ID, Hours, Impacts (n) and Impacts (g).

As shown in Fig. 2, the traumatic brain injury indicator database 10 is further configured with algorithmic means for analysing data in the profile record to determine whether a second alert criteria is met, the second alert criteria relating to cumulative head injury or mTBI risk for the participant.

The algorithmic means may be provided by an artificial intelligence (Al) driven algorithm and/or machine learning algorithm, such as a K-Nearest Neighbour Machine Learning Algorithm executing module 25, and a Long-Short-Term Memory deep learning algorithm module 26.

A profile record 20 for the participant 2 is configured with historical data, including impact force data and physiological data recorded for previous head injury risk events and personal characteristics including biomarkers and other physiological data relating to the participant 2. The traumatic brain injury indicator database 10 comprises means 28 to add, delete and edit a participant profile record 20, together with a module 29 to fetch data from the database 10 with which to populate fields of a profile record 20.

The traumatic brain injury indicator database 10 is also configured with data records corresponding to histories of clinically validated injured and non-injured participants.

The system 1 further includes means 19 to analyse data in the traumatic brain injury indicator database 10 coupled with means 27 to compare data in the participant's profile record with a second alert criteria threshold level computed from the records of data for clinically validated injured and non-injured participants. The second alert criteria indicate an updated elevated risk of cumulative head injury or mTBI.

If the second alert criteria threshold level is equalled or exceeded then a second alert criteria is deemed to be met and a substantially instantaneous alert is transmitted to the at least one remote computing device 7.

On receiving an alert interventions may be considered by coaches and medical staff 11, such as removing the participant 2 from the field of play

Indicated by the reference numeral 30, is a module for analysing outcomes which serves as a feedback loop to continuously improve the performance and accuracy of the system 1.

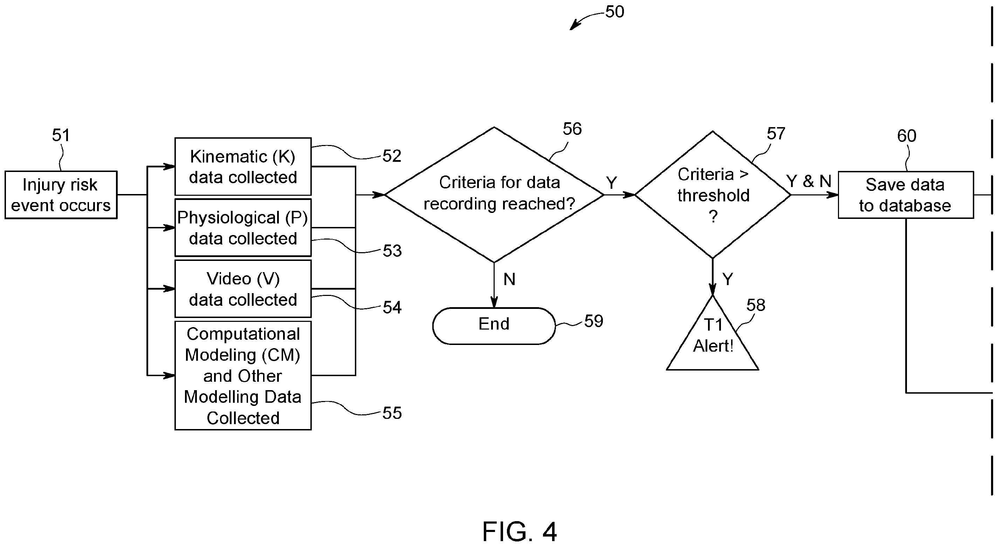

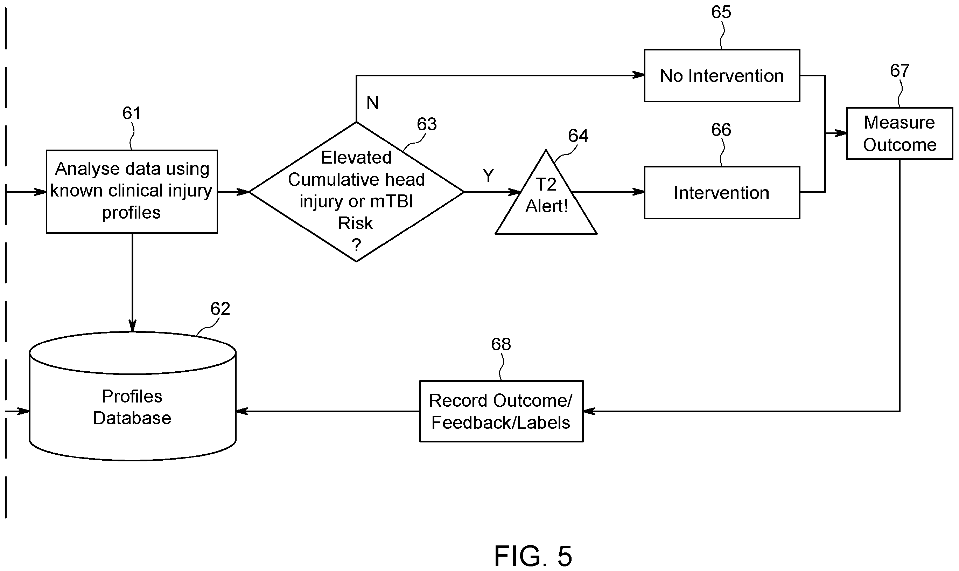

Figs. 4 and 5 are process flow diagrams showing steps in a method 50 configured according to the invention.

At step 51, an injury risk event occurs that generates data, including, at step 52, impact force data or kinematic data from at least one biomechanical sensor 3 and, at step 53, physiological data from a physiological sensor 3. Video data related to the injury risk event may optionally be collected at step 54, together with computational modelling data and other modelling data at step 55.

The data obtained at steps 52, 53, 54 and 55 are assessed at step 56 to determine whether a preliminarily criteria for recording the data is met. If the data recording criteria is not met then the process ends at step 59.

Conversely, if the data recording criteria is met then at step 57 the impact force data and physiological data (and other data obtained) is compared with a first alert criteria threshold level. If the first alert criteria threshold level is equalled or exceeded then a first alert criteria is determined to be met and, at step 58, an instantaneous alert is outputted. If the first alert criteria threshold level is not at least equalled, then an alert is not outputted.

At step 60 the impact force data and physiological data (and other data obtained) is saved to a participant profile in a traumatic brain injury indicator database 62.

As shown at step 61, data for a participant's record in the traumatic brain injury indicator database 62 is analysed with clinically validated injured and non-injured participants using machine learning and artificial intelligence methods, and step 63, determines whether a second alert criteria threshold level for an elevated risk is met.

If the second alert criteria threshold level is met then, at step 64, an alert is outputted and at step 66, interventions considered.

Conversely, if the second alert criteria threshold level for an elevated risk is not met then an alert is not outputted and, at step 65, no intervention is necessary.

At step 67, outcomes are measured and analysed and serve as a feedback loop, as shown at step 68, to thereby continuously improving system performance and accuracy.

The present invention provides a system and method that avoids delays in participants receiving potentially lifesaving interventions following brain injury events thereby decreasing the risk of long-term damage from repeated, undiagnosed head impacts.

By its use at least one biomechanical sensor and at least one physiological sensor the present invention provides advanced monitoring systems that can detect and alert teams, coaches and staff to potential injuries in near real time. Advanced analytics including machine learning algorithms are used to provide near real-time detection and prediction of mTBI by processing the multimodal data and the cumulative effect of head impacts from clinical data with components, including headgear with associated sensors, data collection and processing with cloud storage and advanced analytics platform using machine learning and Al.

The present invention correlates sensor data (real-time and historical) with a scientifically derived traumatic brain injury (TBI) indicator database via a proprietary predictive Al algorithm that is configured to output alerts indicating that participant should seek medical attention if a "concussion" or mTBI is suspected.

The at least one biomechanical sensor and at least one physiological sensor are configured to continuously transmit data in substantially instantaneously / near real-time to a digital health data management platform, which accesses a clinically validated library of player profiles and impacts and clinically validated indicator data sets.

The system and method of the present invention transmits alerts to team management, medical staff or parents to risk of brain injuries resulting from either a large single impact or repeated/sub-concussive impacts with reference to participants own personalised impact and biomarker data.

It is to be understood that the invention is not limited to the specific details described herein which are given by way of example only and that various modifications and alterations are possible without departing from the scope of the invention.

## Claims

1. A system configured for identifying, monitoring, predicting and/or detecting brain injury to a human participant in an active environment, the system comprising:
at least one physiological sensor communicatively coupled to the participant to measure one or more physiological characteristics of the participant, the at least one physiological sensor configured to transmit data relating to the measured one or more physiological characteristics as physiological data,
at least one biomechanical sensor communicatively coupled to the participant to measure impact forces experienced by the participant, the at least one biomechanical sensor configured to transmit data relating to the measured impact forces as impact force data,
means for processing the impact force data and physiological data to determine whether a first alert criteria for a head injury risk event is met, and
means for outputting a substantially instantaneous alert or notification to one or more remote computing devices if the first alert criteria is met.

2. The system as claimed in Claim 1, in which the at least one biomechanical sensor is one or more or a combination of: a force sensitive resistor sensor, an accelerometer sensor and/or a gyroscope sensor.

3. The system as claimed in Claim 1 or Claim 2, in which the at least one physiological sensor is one or more or a combination of: an Electroencephalography (EEG) sensor, a Transcranial Doppler (TCD) sensor, an electrocardiography (ECG) sensor, a Photoplethysmography (PPG) sensor, a Galvanic skin response (GSR) sensor, a heart rate sensor and derivatives, and/or a body temperature sensor.

4. The system as claimed in any one of the previous Claims, in which the at least one physiological sensor and the at least one biomechanical sensor are integrated in headgear worn by the participant.

5. The system as claimed in any one of the previous Claims, in which the means for processing the impact force data and physiological data comprises means for comparing the impact force data and physiological data with a first alert criteria threshold level, whereby if the impact forces corresponding to the impact force data and the physiological characteristics corresponding to the physiological data are determined to be equal to or exceed the first alert criteria threshold level then the first alert criteria is determined to be met.

6. The system as claimed in any one of the previous Claims, further comprising a traumatic brain injury indicator database configured with a profile record for the participant, and the impact force data and physiological data are recorded in the profile record for the participant.

7. The system as claimed in Claim 6, in which the traumatic brain injury indicator database is configured with algorithmic means for analysing data in the profile record to determine whether a second alert criteria is met, the second alert criteria relating to an updated cumulative head injury or mild traumatic brain injury risk for the participant.

8. The system as claimed in Claim 6 or Claim 7, in which the profile record for the participant is configured with historical data, including impact force data and physiological data recorded for previous head injury risk events and personal characteristics including biomarkers and other physiological data relating to the participant.

9. The system as claimed in any one of Claims 6 to 8, the system further comprising means to compare data in the participant's profile record with a second alert criteria threshold level computed from records of data for clinically validated injured and non-injured participants, whereby if the second alert criteria threshold level is equalled or exceeded then a second alert criteria relating to an updated elevated risk of cumulative head injury or mTBI is deemed to be met and a substantially instantaneous alert notification is transmitted to the at least one remote computing device.

10. A method configured for identifying, monitoring, predicting and/or detecting brain injury to a human participant in an active environment, the method comprising steps of:
measuring one or more physiological characteristics of the participant by at least one physiological sensor communicatively coupled to the participant and transmitting data relating to the measured one or more physiological characteristics as physiological data,
measuring impact forces experienced by the participant by at least one biomechanical sensor communicatively coupled to the participant and transmitting data relating to the measured impact forces as impact force data,
processing the impact force data and physiological data to determine whether a first alert criteria for a head injury risk event is met, and
outputting a substantially instantaneous alert notification to one or more remote computing devices if the first alert criteria is met.

11. A method as claimed in Claim 10, in which the step of processing the impact force data and physiological data comprises comparing the impact force data and physiological data with a first alert criteria threshold level, whereby if the impact forces corresponding to the impact force data and the physiological characteristics corresponding to the physiological data are determined to be equal to or exceed the first alert criteria threshold level then the first alert criteria is determined to be met.

12. The method as claimed in Claim 10 or Claim 11, the method comprising a step of configuring a traumatic brain injury indicator database with a profile record for the participant, in which the impact force data and physiological data are recorded in the profile record.

13. The method as claimed in Claim 12, comprising a further step of analysing data in the profile record using algorithms to determine whether a second alert criteria is met, the second alert criteria relating to an updated cumulative head injury or mild traumatic brain injury risk for the participant.

14. The method as claimed in Claim 12 or Claim 13, comprising a further step of configuring the profile record for the participant with historical data, including impact force data and physiological data recorded for previous head injury risk events and personal characteristics including biomarkers and other physiological data relating to the participant, and the traumatic brain injury indicator database with data records corresponding to histories of clinically validated injured and non-injured participants.

15. The method as claimed in any one of Claims 12 to 14, comprising a further step of comparing data in the participant's profile record with a second alert criteria threshold level computed from records of data for clinically validated injured and non-injured participants, whereby if the second alert criteria threshold level is equalled or exceeded then a second alert criteria relating to an updated cumulative head injury or mild traumatic brain injury risk for the participant is determined to be met and a substantially instantaneous alert is transmitted to the at least one remote computing device.
